# EUROPEAN PATENT APPLICATION

(11) **EP 4 008 366 A1**
(43) Date of publication of application: **08.06.2022**
(21) Application number: 20211356.9
(22) Date of filing: 02.12.2020
(51) Int. Cl.: A61L 9/20

(54) **DEVICE FOR THE STERILISATION OF A GAS**

(71) Applicant: Heraeus Noblelight GmbH, 63450 Hanau (DE)
(72) Inventor: WEIß, Oliver, 63801 Kleinostheim (DE); THIEL, Johannes, 63450 Hanau (DE); GRAZIEL, Bernhard, 63801 Kleinostheim (DE); SCHÄFER, Andreas, 63801 Kleinostheim (DE); SCHULZ, Dominik, 64760 Oberzent (DE); BRENTRUP, Berthold, 64760 Oberzent (DE); LEHMANN, Lutz, 64760 Oberzent (DE); BANGERT, Lukas, 64760 Oberzent (DE); LÖB, Daniel, 64743 Oberzent (DE)
(74) Representative: Herzog IP Patentanwalts GmbH

(57) **Abstract**

A device comprising
a.) an electronics unit (101);
b.) an inner longitudinal compartment (102), wherein the inner longitudinal compartment (102) is adapted and arranged to allow the circulation of a gas through the inner longitudinal compartment (102);
c.) at least one lighting means (103), wherein the at least one lighting means (103) is mounted inside the inner longitudinal compartment (102);
d.) at least one circulation means (104), adapted and arranged for the circulation of the gas;
e.) a housing (105), wherein the housing (105) at least partially encloses
i.) the electronics unit (101),
ii.) the inner longitudinal compartment (102) with the at least one lighting means (103),
iii.) the at least one circulation means (104);

wherein
the inner longitudinal compartment (102) is adapted and arranged to be removable from, and insertable into, the housing (105).

## Description

### FIELD OF THE INVENTION

The invention pertains to a device comprising
a.) an electronics unit;
b.) an inner longitudinal compartment, wherein the inner longitudinal compartment is adapted and arranged to allow the circulation of a gas through the inner longitudinal compartment;
c.) at least one lighting means, wherein the at least one lighting means is mounted inside the inner longitudinal compartment;
d.) at least one circulation means, adapted and arranged for the circulation of the gas;
e.) a housing, wherein the housing at least partially encloses
   i.) the electronics unit,
   ii.) the inner longitudinal compartment with the at least one lighting means,
   iii.) the at least one circulation means;
wherein the inner longitudinal compartment is adapted and arranged to be removable from, and insertable into, the housing. The invention also pertains to a process for producing a gas using said device.

### BACKGROUND OF THE INVENTION

Devices for the sterilisation of a gas using UV light are well-known in the art. Such devices are widely used, for example in food processing plants, stables for breeding mammals and avians, and warehouses. The devices are very often also used for extended periods of time, such as multiple years. Examples of such devices include the devices of Sterilair AG (Switzerland) and the UVpro devices of orca GmbH (Germany). Devices, such as the aforementioned prior art, have the disadvantage that not only do the devices require a significant amount of downtime for maintenance and service, but there is also a very large safety risk for the operators involved in the servicing and maintenance. Furthermore, these devices cannot easily be adapted for different requirements. If the requirements of a customer changes, e.g., the customer requires a device with a smaller rate of circulation through the device, this almost always requires a customer to purchase a new device. Additionally, the devices currently available for purchase are also limited in their ability to sterilise a gas.

### OBJECTS OF THE INVENTION

An object of the present invention is to at least partially overcome at least one of the disadvantages encountered in the state of the art.

It is a further object of the invention to provide a device for the sterilisation of a gas that increases the safety of operator during maintenance and servicing of said device.

It is a further object of the invention to provide a device for the sterilisation of a gas that has reduced downtime.

It is a further object of the invention to provide a device for the sterilisation of a gas that reduces the time required to replace a component, e.g., a circulation means or inner longitudinal compartment, of said device.

It is a further object of the invention to provide a device for the sterilisation of a gas that increases the efficacy of the device, in particular the reduction of a concentration of a micro-organism.

It is a further object of the invention to provide a device for the sterilisation of a gas that can be cleaned more easily.

It is a further object of the invention to provide a device for the sterilisation of a gas that can be more easily customised for different operating conditions e.g., different room sizes, or a different light intensity of a lighting means of said device.

It is a further object of the invention to provide a device for the sterilisation of a gas that can be more easily sterilised.

It is a further object of the invention to provide a device for the sterilisation of a gas that can be used in clean rooms.

It is a further object of the invention to provide a device for the sterilisation of a gas that can be used in rooms comprising a micro-organism that is hazardous to either mammal health, in particular human health, avian health, reptile health, or two or more thereof.

### PREFERRED EMBODIMENTS

A contribution to at least partially fulfilling at least one of the above-mentioned objects is made by the independent embodiments. The dependent embodiments provide preferred embodiments which contribute to at least partially fulfilling at least one of the objects.
|1| A device comprising
   a.) an electronics unit;
   b.) an inner longitudinal compartment, wherein the inner longitudinal compartment is adapted and arranged to allow the circulation of a gas through the inner longitudinal compartment;
   c.) at least one lighting means, wherein the at least one lighting means is mounted inside the inner longitudinal compartment;
   d.) at least one circulation means, adapted and arranged for the circulation of the gas;
   e.) a housing, wherein the housing at least partially encloses
      i.) the electronics unit,
      ii.) the inner longitudinal compartment with the at least one lighting means,
      iii.) the at least one circulation means;
   wherein
   the inner longitudinal compartment is adapted and arranged to be removable from, and insertable into, the housing.
|2| The device according to embodiment |1|, wherein the electronics unit is adapted and arranged to be removable from, and insertable into, the housing.
|3| The device according to any of the preceding embodiments, wherein the housing comprises at least one lid adapted and arranged to allow the circulation of the gas through the at least one lid.
   In an aspect of embodiment |3|, it is preferred that the device comprises at least two lids. In this aspect, it is preferred that the at least two lids are arranged at opposite ends of the inner longitudinal compartment.
   In an aspect of embodiment |3|, it is preferred that the at least one lid is adapted and arranged to reduce the intensity of the light, emitted by the at least one lighting means, that escapes from the inner longitudinal compartment. In this aspect, it is preferred that the at least on lid reduces the intensity of the escaping light by at least 50 %, more preferably by at least 70 %, and further preferably by at least 80 %. It is preferred that the reduction in the intensity of the escaping light is measured with respect to a device, preferably according to the present invention, that does not have a lid.
   In a further aspect of embodiment |3|, it is preferred that the at least one lid is adapted and arranged to form a seal, more preferably a gastight seal. In this aspect, it is preferred that the seal reduces the amount of gas that can escape between the at least one lid and the inner longitudinal compartment.
|4| The device according to any of the preceding embodiments, wherein the circulation means is adapted and arranged to be removable from, and insertable into, the housing.
|5| The device according to any of the preceding embodiments, wherein at least one or all of the following is slidably removable from, and insertable into, the housing:
   a.) the inner longitudinal compartment;
   b.) the electronics unit;
   c.) the at least one circulation means;
   d.) the at least one lid.
   For embodiment |5|, all possible combination of the features a. to d. are preferred aspects of the embodiment. These combinations are e.g., a; b; c; d; a, b; a, c; a, d; b, c; b, d; c, d; a, b, c; a, b, d; a, c, d; b, c, d; a, b, c, d. In an aspect of embodiment |5|, it is particularly preferred that the inner longitudinal compartment is slidably removable.
|6| The device according any of the preceding embodiments, wherein at least one or all of the following is adapted and arranged to engage the housing using at least one mechanical fit:
   a.) the inner longitudinal compartment;
   b.) the electronics units;
   c.) the at least one circulation means;
   d.) the at least one lid.
   For embodiment |6|, all possible combination of the features a. to d. are preferred aspects of the embodiment. These combinations are e.g., a; b; c; d; a, b; a, c; a, d; b, c; b, d; c, d; a, b, c; a, b, d; a, c, d; b, c, d; a, b, c, d.
|7| The device according to the preceding embodiment |6|, wherein the mechanical fit is selected from the group consisting of a clearance fit, a transitional fit, an interference fit, and a snap fit.
   In an aspect of embodiment |7|, it is particularly preferred that the at least on lid engages the housing using a snap fit.
|8| The device according to any of the preceding embodiments, wherein at least one or all of the following is adapted and arranged to restrict a movement of the inner longitudinal compartment along at least one direction to less than 5 cm, preferably to less than 3 cm, and more preferably to less than 1 cm:
   a.) the at least one lid;
   b.) the at least one mechanical connector;
   c.) at least one rail.
   For embodiment |8|, all possible combination of the features a. to c. are preferred aspects of the embodiment. These combinations are e.g., a; b; c; a, b; a, c; b, c; a, b, c.
|9| The device according to any of the preceding embodiments, wherein at least 30 %, preferably at least 40 %, and more preferably at least 50 % of a surface of an outer wall of the inner longitudinal compartment does not touch a surface of an inner wall of the housing.
|10| The device according to any of the preceding embodiments, wherein a distance between at least one section of the inner wall of the housing and at least one section of the outer wall of the inner longitudinal compartment is in the range of 10 mm to 100 mm, preferably in the range of 20 mm to 90 mm, and more preferably in the range of 30 mm to 80 mm.
   In an aspect of embodiment |10|, it is preferred that the distance is the minimum distance between the inner wall of the housing and the outer wall of the inner longitudinal compartment.
|11| The device according to any of the preceding embodiments, wherein a total inner volume of the housing is in the range of 1.1 to 3.0 times, preferably in the range of 1.2 to 2.8 times, and more preferably in the range of 1.5 to 2.5 times larger than a total volume of the inner longitudinal compartment.
   In an aspect of embodiment |11|, it is preferred that the total inner volume of the housing is a volume enclosed by the housing. In a further aspect of embodiment |11|, it is preferred that a total volume of the inner longitudinal compartment is a volume enclosed by the inner longitudinal compartment.
|12| The device according to any of the preceding embodiments, wherein the at least one lighting means is adapted and arranged to emit light with a peak wavelength in the range of 10 nm to 1050 nm, preferably in the range of 100 nm to 900 nm, more preferably in the range of 180 nm to 700 nm, even more preferably in the range of 200 nm to 400 nm, further preferably in the range of 200 nm to 300 nm, and even further preferably in the range of 240 nm to 280 nm.
   In an aspect of embodiment |12|, it is preferred that the light is emitted as a spectrum, more preferably a spectrum with a global maximum. If the spectrum has a global maximum, it is preferred that the global maximum of the spectrum is taken as the peak wavelength. In this aspect it is preferred that the global maximum is the wavelength where most of the energy of the light is emitted. In another aspect of embodiment |12|, it is preferred that the light is emitted as an emission line. In this aspect it is preferred that the emission line is taken as the peak wavelength.
|13| The device according to any of the preceding embodiments, wherein the inner longitudinal compartment has a total volume in the range of 0.01 m³ to 0.2 m³, preferably in the range of 0.04 m³ to 0.15 m³, and more preferably in the range of 0.06 m³ to 0.1 m³.
   In an aspect of embodiment |13|, it is preferred that a total volume of the inner longitudinal compartment is a volume enclosed by the inner longitudinal compartment.
|14| The device according to any of the preceding embodiments, wherein at least one or all of the following applies:
   a.) the rate of circulation through the device is in the range of 400 m³/h to 2500 m³/h, preferably in the range of 500 m³/h to 1900 m³/h, and more preferably in the range of 900 m³/h to 1200 m³/h;
   b.) the at least one lighting means has an intensity in the range of 10 mW/cm² to 500 mW/cm², preferably in the range of 20 mW/cm² to 400 mW/cm², and more preferably in the range of 30 mW/cm² to 300 mW/cm².
   In an aspect of embodiment |14|, it is preferred that the intensity of the at least one lighting means is the total intensity of all the lighting means in the inner longitudinal compartment. E.g., if the inner longitudinal compartment has three lighting means, the intensity is the sum of the individual intensities of the three lighting means.
   For embodiment |14|, all possible combination of the features a. and b. are preferred aspects of the embodiment. These combinations are e.g., a; b; a, b.
|15| The device according to any of the preceding embodiments, wherein an inner wall of the inner longitudinal compartment is adapted and arranged to reflect light.
   In an aspect of embodiment |15|, it is preferred that a surface of an inner wall of the inner longitudinal compartment is a polished surface. In another aspect of embodiment |15|, it is preferred that the inner wall of the longitudinal compartment comprises a material that has a maximum reflectivity at the peak wavelength of the light emitted by the lighting means.
|16| The device according to any of the preceding embodiments, wherein at least one or all of the following applies:
   a.) a cross-section of the inner longitudinal compartment is circular;
   b.) the inner longitudinal compartment has a length in the range of 500 mm to 1600 mm, preferably in the range of 600 mm to 1400 mm, and more preferably in the range of 700 mm to 1200 mm;
   c.) the inner longitudinal compartment has a width in the range of 50 mm to 750 mm, preferably in the range of 150 mm to 600 mm, and more preferably in the range of 250 mm to 450 mm.
   For embodiment |16|, all possible combination of the features a. to c. are preferred aspects of the embodiment. These combinations are e.g., a; b; c; a, b; a, c; b, c; a, b, c.
|17| The device according to any of the preceding embodiments, wherein at least one or all of the following applies:
   a.) the inner longitudinal compartment comprises a metal, preferably aluminium;
   b.) the housing comprises a metal, preferably stainless steel.
   For embodiment |17|, all possible combination of the features a. and b. are preferred aspects of the embodiment. These combinations are e.g., a; b; a, b.
|18| A room comprising the device according to any of the preceding embodiments |1| to |17| and a micro-organism, wherein a concentration of the microorganism in the room is less than 10⁴ PFU/m³, preferably less than 10³ PFU/m³, more preferably less than 10² PFU/m³, and further preferably less than 10¹ PFU/m³.
   In an aspect of embodiment |18|, it is preferred that the micro-organism includes at least one or all of the following: bacteria, viruses, protozoa, and fungi, such as yeast and mould. In this aspect, it is preferred that the micro-organism is harmful to a mammal, more preferably a human, an avian, a reptile, or two or more thereof.
   In an aspect of embodiment |18|, it is preferred that a ceiling of the room has a height of at least 3 m, more preferably at least 3.2 m, even more preferably at least 3.5 m, and further preferably at least 4 m.
|19| The room according to the preceding embodiment |18|, wherein the room has a total volume that is at least 300 times, preferably at least 400 times, and more preferably at least 500 times larger than the total volume of the inner longitudinal compartment.
|20| The room according to any of the preceding embodiments |18| to |19|, wherein the room is selected from the group consisting of a storage room, an assembly room, a processing room, a manufacturing room, a room for congregation, and a room for treating at least one medical condition.
   In an aspect of embodiment |20|, it is preferred that the room is used for at least one or all of the following: storing items, e.g., food items, curing food items, processing items, e.g., food items. In this aspect it is preferred that the food items are preferably for consumption by a mammal, more preferably a human. In an aspect of embodiment |20|, an example of an assembly room is a room for assembling a device, e.g., an electronic device. In an aspect of embodiment |20|, an example of a manufacturing room is a room for manufacturing a device, e.g., an automobile. In an aspect of embodiment |20|, examples of a room for congregation are a foyer of a public building, a train station, churches, city halls, concert halls, auditoriums. In an aspect of embodiment |20|, an example of a medical condition is a disease caused by a micro-organism.
|21| A process for producing a gas, comprising as process steps
   a.) providing a device according to any of the preceding embodiments |1| to |17|;
   b.) operating the device by feeding a first gas through the inner longitudinal compartment to obtain the gas.
   In an aspect of embodiment |21|, it is preferred that a concentration of a micro-organism in the gas is at least 30 %, more preferably at least 50 %, further preferably at least 70 %, and particularly preferably at least 90 % lower than a concentration of the micro-organism in the first gas.
|22| The process according to embodiment |21|, wherein the gas has a concentration of the micro-organism that is less than 10⁴ PFU/m³, preferably less than 10³ PFU/m³, more preferably less than 10² PFU/m³, and further preferably less than 10¹ PFU/m³.
|23| The process according to any of the embodiments |21| to |22|, wherein the inner longitudinal compartment is replaced at least once by a further inner longitudinal compartment.

In an aspect of embodiment |23|, it is preferred to replace the inner longitudinal compartment by the further inner longitudinal compartment during maintenance, servicing, or both, of the inner longitudinal compartment.

### FURTHER DESCRIPTION

### Circulation of the gas

If a component, e.g., an inner longitudinal compartment, of the device according to the present invention is adapted and arranged to allow for the "*circulation*" of a gas, this should be understood to mean that a gas can flow through the component. In an aspect of the invention, it is preferred that the gas can circulate in any direction though a component. In a further aspect of the invention, a preferred gas is air, e.g., the Earth's atmosphere.

### Removable and insertable

In an aspect of the invention, it is preferred that at least one component, e.g., the inner longitudinal compartment, is removable from, and insertable into, the housing. In this aspect it is preferred that the at least one component can be removed, inserted, or both, without the use of at least one tool. In another aspect of the invention, a housing of a device, according to the present invention, is fastened to at least one or all of the following using at least one fastening means: a floor, a wall, a ceiling, a roof. In this aspect, it is preferred that the at least one component can be removed, inserted, or both, without requiring that the at least one fastening means should be loosened. E.g., the housing of the device is fastened to two cables, wherein the two cables are fastened to a ceiling of a storage room. The device therefore hangs from the ceiling of the storage room. The inner longitudinal compartment can be removed from the housing without requiring that the housing should be taken down from the ceiling.

In an aspect of the invention, it is preferred that a component that is removable from, and insertable into the housing, is not fastened to the housing. In a further aspect of the invention, it is preferred that a component that is removable from, and insertable into, the housing is adapted and arranged to move with respect to the housing, when the component is at least partially enclosed in the housing.

### Slidably removable and insertable

In an aspect of the invention, it is preferred that at least one component, e.g., the inner longitudinal compartment, is slidably removeable from, and insertable into, the housing. In this aspect, it is preferred that the slidably removable and insertable component is adapted and arranged to move along a preferred direction when the component is at least partially enclosed in the housing. In this aspect, it is preferred that the work required to move the slidably removable and insertable component a distance along the preferred direction is less than the work required to move the slidably removable and insertable component the same distance along any other direction. Here work refers to the physical quantity defined as the product of force and distance moved.

In an aspect of the invention, it is preferred that the device according to the present invention comprises at least one sliding means for, e.g., removing an inner longitudinal compartment in a "*slidable manner*" from the housing. Such sliding means are well-known to the skilled person, e.g., rails, at least one wheel adapted and arranged to move along a rail, or a combination thereof.

In an aspect of the invention, it is preferred that the at least one sliding means comprises at least one or all of the following: a plastic, a metal, fibre glass, a composite material, or a combination thereof. Examples of a preferred metal include aluminium and stainless steel, with stainless steel particularly preferred.

### Types of mechanical fit

A *"clearance fit", "transition fit",* and *"interference fit"* should be understood as defined within the classification of the International Organisation for Standardization (ISO). A preferred *"transition fit"* is a fixed fit. A preferred snap fit is an annular snap-fit, a cantilever snap-fit, a torsional snap-fit, or a combination of two or more thereof.

### Inner longitudinal compartment

In an aspect of the invention, it is preferred that a cross-sectional cut of the inner longitudinal compartment has at least one of the following shapes: circular, elliptical, square, rectangular, parallelogram, triangular, a polygon, e.g., a pentagon, a hexagon, or a heptagon. In this aspect, it is more preferred that the cross-sectional cut is circular. In an aspect of the invention, it is preferred that an area of the cross-sectional cut of the inner longitudinal compartment changes along a length of the inner longitudinal compartment. It is preferred that the cross-sectional cut is made perpendicular to a length of the inner longitudinal compartment. In another aspect of the invention, it is preferred that the inner longitudinal compartment comprises at least one mounting means adapted and arranged for mounting the lighting means inside the inner longitudinal compartment. In this aspect, it is preferred that the mounting means is fastened to an inner wall of the inner longitudinal compartment. E.g., moving the inner longitudinal compartment causes the at least one mounting means to move along with the inner longitudinal compartment. Mounting means suitable for the present invention are well-known to the skilled person.

### Electronics unit

In an aspect of the invention, it is preferred that the electronics unit comprises at least one or all of the following: at least one processing unit, at least one printed circuit board, at least one storage medium, at least one means for transmitting and receiving electromagnetic radiation, e.g., an antenna, at least one sensor. In an aspect of the invention, it is preferred that at least one physical quantity is controlled, monitored, regulated, or a combination thereof, by the electronics unit. Examples of the at least one physical quantity are an intensity of the at least one lighting means, a voltage, a current, an airflow though the inner longitudinal compartment, a number of revolutions of the circulation means, an uptime of the at least one lighting means, a temperature of the device.

In an aspect of the invention, it is preferred that at least 30 %, preferably at least 40 %, and more preferably at least 50 % of a surface of an outer wall of the inner longitudinal compartment does not touch the electronics unit.

### Circulation means

In an aspect of the invention, it is preferred that the circulation comprises at least one fan. Examples of a preferred fan include an axial fan, a centrifugal fan, and a cross-flow fan, with an axial fan particularly preferred. Circulation means suitable for the present invention are well-known to the skilled person.

### Lighting means

In an aspect of the invention, it is preferred that the device comprises at least two lighting means, or at least three lighting means. In another aspect of the invention, it is preferred that the at least one lighting means is adapted and arranged for being a germicidal lamp. In a further aspect of the invention, it is preferred that the at least one lighting means is at least one of the following: a low-pressure mercury lamp, a high-pressure mercury lamp, an excimer lamp, and a semi-conductor light source. A preferred semi-conductor light source is a light emitting diode. Lighting means suitable for the present invention are well-known to the skilled person, and can be obtained from, e.g., Heraeus Noblelight GmbH (Germany), such as the UV Lamp NNI 370/77 XL HR.

### Lid

In one aspect of the invention, it is preferred that a lid comprises a plurality of slits. In this aspect, it is preferred that the lid comprises a plurality of lamellas. In another aspect of the invention, it is preferred that the lid comprises a plurality of holes. In this aspect, it is preferred that the plurality of holes are arranged in a honeycomb-structure, a grid, or a combination thereof. In a further aspect of the invention, it is preferred that the plurality of holes, slits, or both, cover at least 70 %, more preferably at least 80 %, and further preferably at least 90 % of a surface of the lid. In another aspect of the invention, it is preferred that the lid comprises at least one or all of the following: a plastic, a metal, fibre glass, a composite material, or a combination thereof. Examples of a preferred metal include aluminium and stainless steel, with stainless steel particularly preferred.

### The housing

In an aspect of the invention, it is preferred that the housing comprises at least one or all of the following: a plastic, a metal, fibre glass, a composite material, or a combination thereof. In this aspect, it is particularly preferred that the housing comprises a metal. Examples of a preferred metal include aluminium and stainless steel, with stainless steel particularly preferred.

In one aspect of the invention, it is preferred that the lid is partially enclosed in the housing. In another aspect of the invention, it is preferred that the lid can be fastened to the housing.

### BRIEF DESCRIPTION OF THE DRAWINGS

The figures serve to exemplify the present invention, and should not be viewed as limiting the invention. Note that the figures are not drawn to scale.

### List of figures

- Fig. 1:: schematic illustration showing a cross-section of the device according to the present invention, viewed from the side.
- Fig. 2:: schematic illustration showing the device according to the present invention, viewed from the front.
- Fig. 3:: diagram showing the steps of a process for producing a gas.

### Description of figures

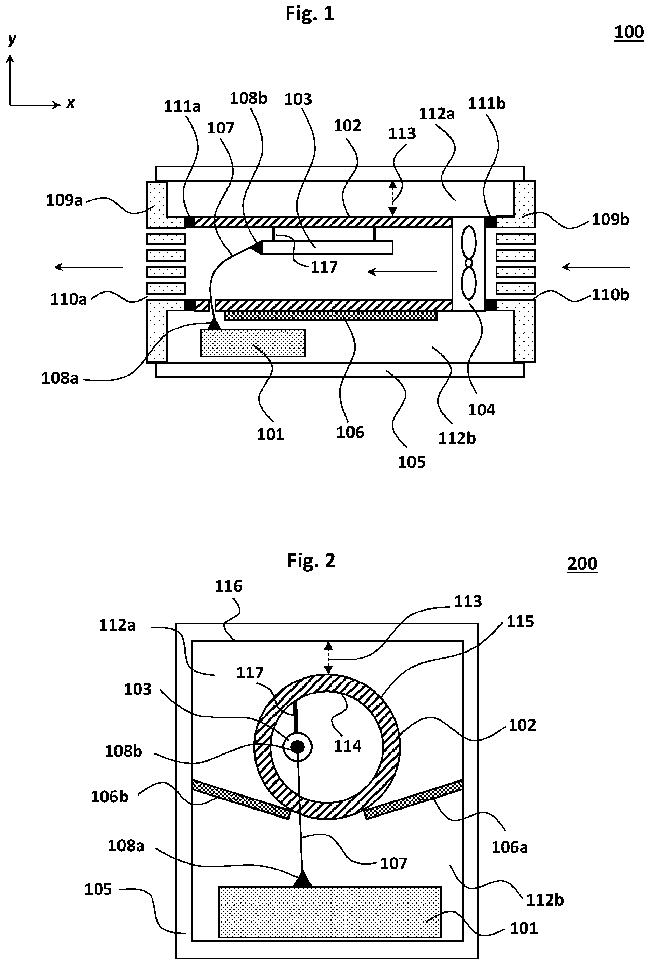

Fig. 1 shows a schematic illustration of cross-section of a device according to the present invention 100, viewed from the side. The device 100 has an electronics unit 101, as well as an inner longitudinal compartment 102. The inner longitudinal compartment 102 is adapted and arranged to allow a gas to flow (circulate) through it, as indicated by the arrows in Fig. 1. Furthermore, the inner longitudinal compartment 102 is adapted and arranged to slide horizontally (i.e., along the x-axis in Fig. 1) along a rail (a sliding means) 106. Both the inner longitudinal compartment 102 and the rail 106 are made of aluminium.

Fig. 1 further shows that mounted inside the inner longitudinal compartment 102 is a lighting means 103. The lighting means 103 is mounted inside the inner longitudinal compartment 102 using a mounting means 117, wherein the mounting means is fastened to the inner longitudinal compartment 102. The lighting means 103 is connected to the electronics unit 101 by means of an electric cable 107. The ends of the electric cable 107 is connected to electrical connectors 108a and 108b. Electrical connector 108a plugs into a socket on the electronics unit 101, while electrical connector 108b plugs into a socket on the lighting means 103. This allows for a simplified connection and disconnection of the electronics unit 101 and the lighting means 103 to each other.

Fig. 1 shows that the device 100 further comprises a circulation means 104 for circulating a gas, from the surrounding environment, through the device 100. The device 100 also has a housing 105 that encloses the electronics unit 101, the inner longitudinal compartment 102, and the circulation means 104. The housing 105 is made of stainless steel.

Fig. 1 also shows that the device 100 has two lids 109a and 109b that are partially enclosed in the housing 105, wherein the two lids are positioned at opposite ends of the inner longitudinal compartment 102. The lids 109a and 109b engage the housing 105 by means of a snap-fit. The lids 109a and 109b have a plurality of holes 110a and 110b, respectively, that allow a gas to flow through the lids 109a and 109b. Between the lid 109b and the circulation means 104 is a seal 111b that prevents the gas that flows into the device from flowing into the hollows 112a and 112b. Between the lid 109a and the inner longitudinal compartment 102 is another seal 111a that also reduces the amount of gas that flows into the hollows 112a and 112b. Fig. 1 also shows a distance 113 between a section of an inner wall of the housing 105 and a section of an outer wall of the inner longitudinal compartment 102.

Fig. 1 also shows that the inner longitudinal compartment 102 is prevented from moving along the x-axis by the lids 109a, 109b, and the circulation means 104. Furthermore, the rail 106 prevents the inner longitudinal compartment 102 from moving along the negative *y*-axis, i.e., downward in Fig. 1. However, when e.g., the lid 109a is removed, the inner longitudinal compartment 102, including the mounting means 117 and the lighting means 103, can be slidably removed along the x-axis from the housing 105.

Fig. 2 shows the device of Fig. 1, viewed from the front. The front of the device 100 in Fig. 1 is defined as the side of the device 100 where the lid 109a is positioned. Furthermore, in Fig. 2 the lid 109a has been removed for illustrative purposes. Fig.2 shows that the inner longitudinal compartment 102 rests on the rails 106a and 106b. Fig. 2 also shows an inner wall 114 and an outer wall 115 of the inner longitudinal compartment 102, as well as an inner wall 116 of the housing 105. A surface of the inner wall 114 of the inner longitudinal compartment 102 is adapted and arranged to reflect the light emitted by the lighting means 103. Fig. 2 also shows that the mounting means 117 is fastened to the inner wall 114 of the inner longitudinal compartment 102.

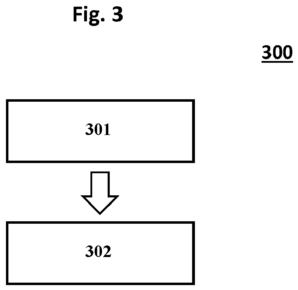

Fig. 3 shows a diagram illustrating the steps of a process for producing a gas 300. In step 301, a device according to Fig. 1 is provided. In step 302, the device is operated by feeding a first gas though the inner longitudinal compartment to obtain a gas. Furthermore, a concentration of a micro-organism is reduced by 95 %, compared to a concentration of the same micro-organism in the first gas.

### EXAMPLES

The invention is illustrated further by way of examples. The invention is not restricted to the examples.

For all examples, the following applies: two rooms with a volume of 350 m³ are provided. Each room has a ceiling that is 4 m high, as measured from a floor of the room. Two devices for the sterilisation of a gas is also provided. The first device, not according to the present invention, is placed in a first room, and the second device, according to the invention, is placed in a second room. Each device is suspended from the ceiling by means of two cables, where each cable has a length of 65 cm. Each of the two cables is fastened to a position on an upper side of the device. Furthermore, each device is suspended parallel to the floor. Each device has a lighting means in the form of a low-pressure mercury lamp that radiates a spectrum of UV-C light with a peak wavelength at 254 nm. Furthermore, each device can circulate 1000 m³/h of gas through the device. In the comparative example and the example, the lighting means is removed and replaced.

### Comparative example

The device of the comparative example is similar to the device of Fig. 1, except that the device of the comparative example does not have an inner longitudinal compartment that can be removed from, and inserted into, the housing. The lighting means is thus mounted inside the housing using a mounting means, wherein the mounting means is fastened to an inner wall of the housing. Furthermore, a surface of an inner wall of the housing is adapted and arranged to reflect the light emitted by the lighting means. The mass of the device of the comparative example is 30 kg.

In order to remove the lighting means, an operator uses a hydraulic platform to reach the device. The operator then detaches the cables from the device. The hydraulic platform is lowered, and after the removing the lid, the lighting means is replaced on the floor of the room. After replacing the lighting means, the device is lifted to the ceiling using the hydraulic platform, where the device is again fastened to the ceiling.

The total time required to detach the device from the ceiling, to replace the lighting means, and to re-fasten the device to the ceiling is 40 minutes.

### Example (according to present invention)

A device according to Fig. 1 is provided. The housing is made from stainless steel, and the inner longitudinal compartment is made of aluminium. The inner longitudinal compartment has a mass of 5 kg.

In order to remove the lighting means, an operator uses a hydraulic platform to reach the device. The operator removes the lid on the side of the device opposite to the side of the device where the circulation means is located. After removing the lid, the operator disconnects the lighting means and the electronics unit by plugging out the electrical cable connecting the lighting means and electronics unit. The operator then removes the inner longitudinal compartment by sliding the inner longitudinal compartment out of the housing along the rail. The hydraulic platform is lowered, and the lighting means is replaced on the floor of the room. After replacing the lighting means, the inner longitudinal compartment is lifted to the ceiling using the hydraulic platform, where the inner longitudinal compartment is slidably inserted into the housing.

The total time required to remove the inner longitudinal compartment from the housing, to replace the lighting means, and to insert the inner longitudinal compartment into the housing is 20 minutes.

Alternatively, instead of inserting the inner longitudinal compartment into the housing after replacing the lighting means, an operator may also replace the inner longitudinal compartment with a further inner longitudinal compartment. The replacement of the inner longitudinal compartment with the further longitudinal compartment can be performed without requiring that the hydraulic platform be lowered.

Further advantages of the present invention are shown in Table 1, which compares the device of the comparative example with the device of the example. The comparative example is taken as the baseline. Table 1 shows that the device of the example provides a significant improvement over the device of the comparative example.

**Table 1: comparison of a device according to the present invention (example) with a device not according to the present invention (comparative example).**

| **Effect** | **Example** | **Comparative example** |
|---|---|---|
| Safety during maintenance and servicing of device | Significantly increased | - |
| Downtime of device | Significantly decreased | - |
| Time required to replace a component | Significantly reduced | - |
| Reduction in micro-organism concentration | Moderately reduced | - |
| Cleaning of reflective surface | Significantly increased | - |
| Customisability of device | Significantly increased | - |
| Sterilisation of device | Increased | - |

The effects of Table 1 are as follows:
- *Safety during maintenance and servicing of device*: the safety of the operators when e.g., replacing a component, such as a lighting means or circulation means.
- *Downtime of device*: the downtime of the device due to maintenance and servicing.
- *Time required to replace a component*: the time required to replace a component, e.g., the lighting means or circulation means
- *Reduction in micro-organism concentration*: an initial concentration of a micro-organism in the rooms of the comparative example and the example is measured. The initial concentration of both rooms is 10⁵ PFU/m³. The devices of the comparative example and the example are allowed to operate for 24 hours. The concentration of the micro-organism is then again measured in the rooms after the 24 hours.
- *Cleaning of reflective surface*: a surface of an inner wall of the housing of the device of the comparative example, as well as a surface of an inner wall of the inner longitudinal comportment of the example, will get coated with dust and films during operation of the devices. This reduces the reflectivity of the inner surface of the housing of the comparative example, as well as the inner surface of the inner longitudinal comportment.
- *Customisability of device*: whether the device can be customised for different rooms and operating conditions, e.g., different room sizes, or different light intensities of the lighting means.
- *Sterilisation of device*: how easy it is to sterilise the device. The devices are often used in clean rooms, or alternatively, rooms with a high concentration of a hazardous micro-organism, where said micro-organism is hazardous to mammal health, avian health, or reptile health. Components of these devices often require replacement. In the case of a clean room, any component or device first has to be sterilised before it can be brought into the room. In the case of a room with a hazardous micro-organism, one has to seal the component or device before one can remove the component or device from the room. The contaminated device or component then has to be transported and sterilised outside the room.

### TEST METHODS

The test methods which follow were utilized within the context of the invention. Unless stated otherwise, the measurements were conducted at an ambient temperature of 23 °C, an ambient air pressure of 100 kPa (0.986 atm), and a relative air humidity of 50 %.

### Peak wavelength of the lighting means

A spectrum of electromagnetic radiation produced by the lighting means is measured using a spectrometer of the type CCS200 from Thorlabs GmbH. The measurement is conducted in accordance with the manufacturer's instructions. The peak wavelength of the measured spectrum is then a local maximum of the spectrum, which is also its global maximum.

### Rate of circulation through device

The rate of circulation through the device is measured using the differential pressure transmitter CP 300 commercially available from Sauermann (Kimo) (France). The differential pressure transmitter is used with a "Debimo" measurement cross.

### Intensity of lighting means

The intensity of the lighting means is measured using a Nobleprobe^{®} measuring device commercially available from Heraeus Noblelight GmbH (Germany). Furthermore, the intensity of the lighting means is measured in the centre of the inner longitudinal compartment. Here the centre is taken with regards to a cross-sectional area of the inner longitudinal compartment, where the cross-sectional area is perpendicular to a length of the inner longitudinal compartment, as well as a centre with regards to the length of the inner longitudinal compartment.

### REFERENCE LIST

- 100: Device
- 101: Electronics unit
- 102: Inner longitudinal compartment
- 103: Lighting means
- 104: Circulation means
- 105: Housing
- 106: Rail
- 107: Electric cable
- 108: Electrical connectors
- 109: Lids
- 110: Plurality of holes in lid
- 111: Seals
- 112: Hollows
- 113: Distance between housing and inner longitudinal compartment
- 114: Inner wall of inner longitudinal compartment
- 115: Outer wall of inner longitudinal compartment
- 116: Inner wall of housing
- 117: Mounting means

- 300: Process for producing a gas
- 301: Provide device according to present invention
- 302: Feeding first gas through inner longitudinal compartment

## Claims

1. A device comprising
a.) an electronics unit (101);
b.) an inner longitudinal compartment (102), wherein the inner longitudinal compartment (102) is adapted and arranged to allow the circulation of a gas through the inner longitudinal compartment (102);
c.) at least one lighting means (103), wherein the at least one lighting means (103) is mounted inside the inner longitudinal compartment (102);
d.) at least one circulation means (104), adapted and arranged for the circulation of the gas;
e.) a housing (105), wherein the housing (105) at least partially encloses
i.) the electronics unit (101),
ii.) the inner longitudinal compartment (102) with the at least one lighting means (103),
iii.) the at least one circulation means (104);
wherein
the inner longitudinal compartment (102) is adapted and arranged to be removable from, and insertable into, the housing (105).

2. The device according to claim 1, wherein the electronics unit is adapted and arranged to be removable from, and insertable into, the housing.

3. The device according to any of the preceding claims, wherein the housing comprises at least one lid adapted and arranged to allow the circulation of the gas through the at least one lid.

4. The device according to any of the preceding claims, wherein the circulation means is adapted and arranged to be removable from, and insertable into, the housing.

5. The device according to any of the preceding claims, wherein at least one or all of the following is slidably removable from, and insertable into, the housing:
a.) the inner longitudinal compartment;
b.) the electronics unit;
c.) the at least one circulation means;
d.) the at least one lid.

6. The device according any of the preceding claims, wherein at least one or all of the following is adapted and arranged to engage the housing using at least one mechanical fit:
a.) the inner longitudinal compartment;
b.) the electronics units;
c.) the at least one circulation means;
d.) the at least one lid.

7. The device according to any of the preceding claims, wherein at least one or all of the following is adapted and arranged to restrict a movement of the inner longitudinal compartment along at least one direction to less than 5 cm:
a.) the at least one lid;
b.) the at least one mechanical connector;
c.) at least one rail.

8. The device according to any of the preceding claims, wherein a total inner volume of the housing is in the range of 1.1 to 3.0 times larger than a total volume of the inner longitudinal compartment.

9. The device according to any of the preceding claims, wherein the at least one lighting means is adapted and arranged to emit light with a peak wavelength in the range of 10 nm to 1050 nm.

10. The device according to any of the preceding claims, wherein at least one or all of the following applies:
a.) the rate of circulation through the device is in the range of 400 m³/h to 2500 m³/h;
b.) the at least one lighting means has an intensity in the range of 10 mW/cm² to 500 mW/cm².

11. The device according to any of the preceding claims, wherein an inner wall of the inner longitudinal compartment is adapted and arranged to reflect light.

12. The device according to any of the preceding claims, wherein at least one or all of the following applies:
a.) a cross-section of the inner longitudinal compartment is circular;
b.) the inner longitudinal compartment has a length in the range of 500 mm to 1600 mm;
c.) the inner longitudinal compartment has a width in the range of 50 mm to 750 mm.

13. The device according to any of the preceding claims, wherein at least one or all of the following applies:
a.) the inner longitudinal compartment comprises a metal;
b.) the housing comprises a metal.

14. A room comprising the device according to any of the preceding claims 1 to 13 and a micro-organism, wherein a concentration of the microorganism in the room is less than 10⁴ PFU/m³.

15. A process for producing a gas, comprising as process steps
a.) providing a device according to any of the preceding claims 1 to 13;
b.) operating the device by feeding a first gas through the inner longitudinal compartment to obtain the gas.
